## Europäisches Patentamt
## European Patent Office
## Office européen des brevets

(11) Publication number: **0 030 132**
**B1**

(12) **EUROPEAN PATENT SPECIFICATION**

(45) Date of publication of patent specification: **16.10.85**

(51) Int. Cl.⁴: **A 61 N 1/36**

(21) Application number: **80304269.6**

(22) Date of filing: **27.11.80**

(54) Cardiac pacemaker with destructible means for output mode selection.

(30) Priority: **28.11.79 US 98159**
**28.11.79 US 98160**

(43) Date of publication of application:
**10.06.81 Bulletin 81/23**

(45) Publication of the grant of the patent:
**16.10.85 Bulletin 85/42**

(84) Designated Contracting States:
**DE GB NL SE**

(56) References cited:
**FR-A-2 133 835**
**FR-A-2 383 674**
**US-A-3 849 638**
**US-A-4 122 547**

(73) Proprietor: **MEDTRONIC, INC.**
**3055 Old Highway Eight P.O. Box 1453**
**Minneapolis Minnesota 55440 (US)**

(72) Inventor: **Thompson, David Lee**
**1660 Onondaga Street N.E.**
**Fridley Minnesota 55432 (US)**
Inventor: **Roline, Glenn Milton**
**4118 - 211th Lane N.W.**
**Anoka Minnesota 55303 (US)**

(74) Representative: **Tomlinson, Kerry John et al**
**Frank B. Dehn & Co. European Patent Attorneys**
**Imperial House 15-19 Kingsway**
**London WC2B 6UZ (GB)**

Courier Press, Leamington Spa, England.

EP 0 030 132 B1

## Description

The invention in general relates to body tissue stimulators and more particularly to a cardiac pacemaker that can be manufactured in a wide variety of models having different output modes, and at the same time is economical to manufacture because all models employ common circuitry.

The art of implantable cardiac pacemakers was first disclosed by Greatbatch in U.S. Patent No. 3,057,356 entitled "Medical Cardiac Pacemaker", which issued in 1962. The device disclosed by Greatbatch included a relaxation oscillator that generated electrical pulses at a fixed rate. These pulses were applied to the heart through a lead to cause the heart to contract each time a pulse occurred.

It became evident early in the development of the art of cardiac pacemakers that it was desirable to design pacemakers so that a range of output parameters could be obtained. For example a variety of pulse rates was desirable because different patients may require different pulse rates, for example children normally require higher pulse rates that adults. As another example, a variety of pulse widths and/or pulse amplitudes was desirable because different patients could have different stimulation thresholds.

In early pacemakers the output parameters were changed by altering individual circuit elements within the pulse circuits. For example the Greatbatch patent mentioned above discloses changing the capacitances and resistances within the circuit to change the pulse rate of the pacemaker. Later improvements disclosed various methods for changing the output parameters, usually involving a means for changing the output parameters of the implanted pacemaker remotely from outside the body. For example, U.S. Patent No. 3,198,195 issued to W. M. Chardack discloses changing the rate and amplitude of the pacemaker pulse by adjusting a potentiometer in the pacemaker with a needlelike tool. U.S. Patent No. 3,311,111 issued to G. L. Blowers discloses using magnetic switches to switch resistors or capacitors in and out of the pacemaker's circuit in order to change the pulse rate of the pacemaker, and U.S. Patent No. 3,518,997 issued to R. W. Sessions discloses a pacemaker with two different oscillating circuits having different output parameters, which circuits can be independently activated by a magnetic switch.

As the pacemaker art developed many improvements were made which made possible a wide variety of output modes in addition to the variable rate and pulse width modes. For example, a sense amplifier was incorporated within the pacemaker circuit in order to provide stimulating pulses only when needed (the demand pacemaker — See U.S. Patent No. 3,478,746 issued to Wilson Greatbatch), and a hysteresis function was added which permits the heart to beat naturally even though it is beating at a slower rate than the rate at which the pacemaker is designed to operate (reissue U.S. Patent No. 28,003, issued to David W. Gobeli). Another improvement to the pacemaker art was the addition of a refractory period in a demand pacemaker. During the refractory period the pacemaker is rendered immune to any signals provided by the sensing amplifier, in order to ensure that the pacemaker stimulation is not suppressed by the sensing of natural electrocardiac signals such as the heart T-wave or spurious electrical signals which occur in the time interval immediately following a stimulated beat of the heart. See U.S. Patent No. 3,433,228 issued to John Walter Keller, Jr. Such improvements greatly increased the variety of output modes that could be incorporated into a pacemaker depending upon the desires and needs of physicians and patients. Other pacemaker improvements added a variety of means and methods whereby the output parameters could be varied. See U.S. Patent Nos. 3,631,860 issued to Michael Lopin; 3,623,486 issued to Barouh V. Berkovits; 3,738,369 issued to Theodore P. Adams and David L. Bowers; 3,713,449 issued to Pieter J. Mulier; and 3,766,928 issued to Herbert E. Goldberg and Jonathon E. Bosworth.

With the advent of a digital pacemaker many improvements were made whereby the output modes of pacemakers could be varied by programming. See U.S. Patents Nos. 3,805,796 issued to Reese S. Terry Jr. and Gomer L. Davies; 3,833,005 issued to Robert C. Wingrove; 4,024,875 issued to James J. Putzke; and 4,066,086 issued to Clifton A. Alferness. All these digital pacemakers were programmed by means of coded radio frequency or other signals applied externally of the patient within whom the pacemaker was implanted.

The manufacturing of pacemakers having a variety of output modes by inserting a variety of different circuit elements had many disadvantages. Insertion of special parts for each mode added complexity to the manufacturing process which was expensive. The modern pacemaker is a highly sophisticated electronic instrument which is very susceptible to degradation of performance by dirt, impurities or other defects. The addition of special circuit elements requires soldering and manipulation of the circuitry which significantly increases the chances of impurities and other minor defects entering the pacemaker. In addition, such addition of elements requires nonstandardized manufacturing operations which can lead to error in selection of parts, and in assembling of parts.

The improvements which permitted the changing of output mode externally to the pacemaker by tools, magnetic fields, or R.F. programming avoided many of the above problems. However, these methods require sophisticated electronics and mechanisms which are expensive to manufacture. Expense is particular not warranted in the large number of cases where the pacemaker needs to be programmed only once in order to adjust it to the particular parameters required by the patient. In addition mechanical adjustment means require a seal about the movable part in the pacemaker, which seal is highly susceptible to failure in the hostile environment of the human body, while the magnetic and R.F. programmed pacemakers may be erroneously programmed due to improper placement or handling of the program device, or may be accidentally reprogrammed by spurious magnetic or electrical signals.

FR—A—2383674 discloses a device for providing electrical stimulation to living tissue, having a pulse generator means with alterable output parameters, and switching means for selecting the output parameters.

According to the invention there is provided a device for providing electrical stimulation to living tissue, comprising a pulse generator having one or more alterable output parameters, said pulse generator comprising a circuit for determining said output parameter(s), characterised in that said pulse generator further comprises destructible means for selecting said output parameter(s), a destructible means input terminal having a first voltage level, a destructible means output terminal, said destructible means being coupled between said destructible means input and output terminals, a transistor having a gate terminal, a source terminal, and a drain terminal, and a strobe means arranged to produce a periodical short pulse coupled to said gate terminal for controlling said transistor, one of said source and drain terminals communicating with an electronic source providing a second voltage level and the other of said source and said drain terminals communicating with said output terminal through a resistance whereby whenever said transistor is turned on by said strobe means while said destructible means is destroyed, said output terminal will assume said second voltage level, and bistable means having an output coupled to an input terminal of said determining circuit and capable of existing in either of two output states and an input communicating with said destructible means output terminal, said output of said bistable means being held in a first output state when said destructible means is intact and said destructible means output terminal is at said first voltage level and said output of said bistable means being held in a second output state when said destructible means is destroyed and said destructible means output terminal is at said second voltage level.

The invention thus enables the provision of a cardiac pacemaker which may be economically manufactured in a wide variety of models having different output modes, which may be easily and accurately programmed to any of a wide variety of output modes, and which can be adjusted to any of a variety of output modes in a manner that presents little risk of contamination of the pacemaker, and of errors in performing such adjustments.

In the practice of the invention a pacemaker is made from components including electronic pulse generating circuitry communicating with at least one output terminal. The circuitry includes destructible means, such as one or more fusible links. The circuitry is adapted so that when there is conduction across the destructible means one output mode is generated, and when the destructible means is destroyed so that there is no conduction across its terminals, another output mode is generated. The destructible means is then destroyed, for example one or more of the fusible links may be fused, to produce a pacemaker having the desired output mode. Preferably the circuitry, except the output terminal, is then sealed within a container that is substantially inert to body fluids so that it may be implanted in a human body.

The apparatus of the invention provides a pulse generator having output mode selection circuitry including a destructible means such as the one or more fusible links. Each destructible means preferably communicates with a mode selection circuit output terminal which is also an input terminal to the portion of the circuitry which determines the output pulse parameters. Preferably a determinate state circuit means also communicates with the terminal to prevent the terminal from floating when the destructible means communicating with the terminal is destroyed. Preferably the output mode selection circuitry is adapted so that the mode selected when there is conduction across the terminals of the destructible means is the safer or more acceptable of the variety of output modes selectable. Thus if after the destructible means have been destroyed there is a failure in the circuitry and conduction is reestablished the mode selected will be safer or more acceptable.

An embodiment of the invention will now be described by way of example and with reference to the accompanying drawings, in which:

FIG. 1 is a block schematic diagram of the pacemaker circuitry for a preferred embodiment of the invention showing the various signals provided between the digital and analog portions of the circuit;

FIG. 2 shows the arrangements of FIGS. 2a and 2b which in turn show, in block format the digital circuitry portion of the pacemaker;

FIG. 3 shows an embodiment of the invention for selecting the output mode and for setting the mode selection output terminals to a determinate electronic state; and

FIG. 4 shows circuitry for selecting the output mode and for setting the mode selection output terminals to a determinate electronic state.

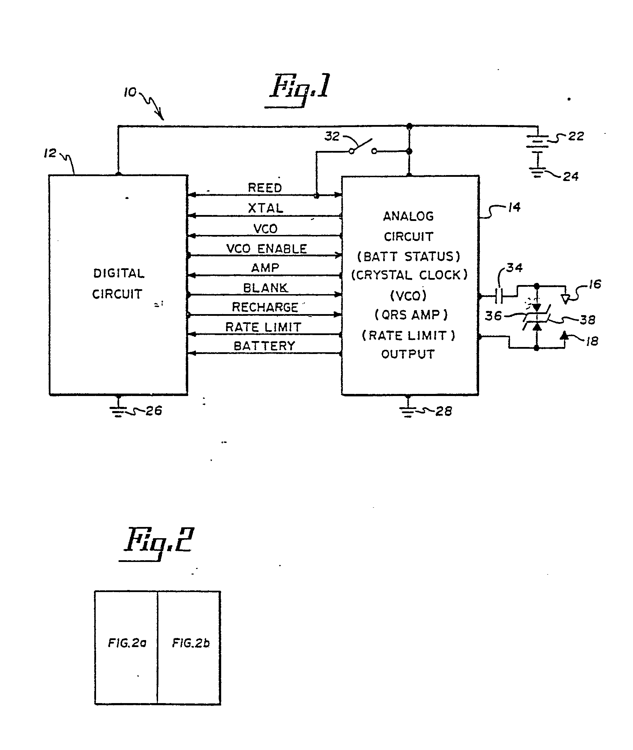

Directing attention to the drawings, FIG. 1 shows a schematic diagram of a cardiac pacemaker circuit. The cardiac pacemaker 10 includes a digital circuit 12 which communicates with an analog circuit 14 to produce an electrical pulse which is delivered via outputs 16 and 18 to leads (not shown) for application to the heart (not shown) to cause the heart to contract. The leads through which the pulses are applied to the heart, the type of pulses applied, and the response of the heart to the pulses is well known in the art and will not be discussed herein.

The embodiment of the pulse generator 10 which shall be described is of the type that is implantable within the human body. However, nothing herein should be construed as limiting the invention described solely to the implantable type pulse generators.

The analog circuit as shown in FIG. 1 consists of a number of generally separate electrical systems. These systems include a battery status monitor, a crystal oscillator clock, a voltage control oscillator clock, a QRS sensing amplifier, a pulse rate limiting circuit and output circuitry which includes a voltage doubler. Each of these analog systems are well known in the art and will not be discussed in detail herein. For a complete description of certain of these circuits reference is made to the following patent applications, commonly owned with the present application: European applications 79302447.2 (EP—A—0011931) and 79302450.6 (EP—A—0011934).

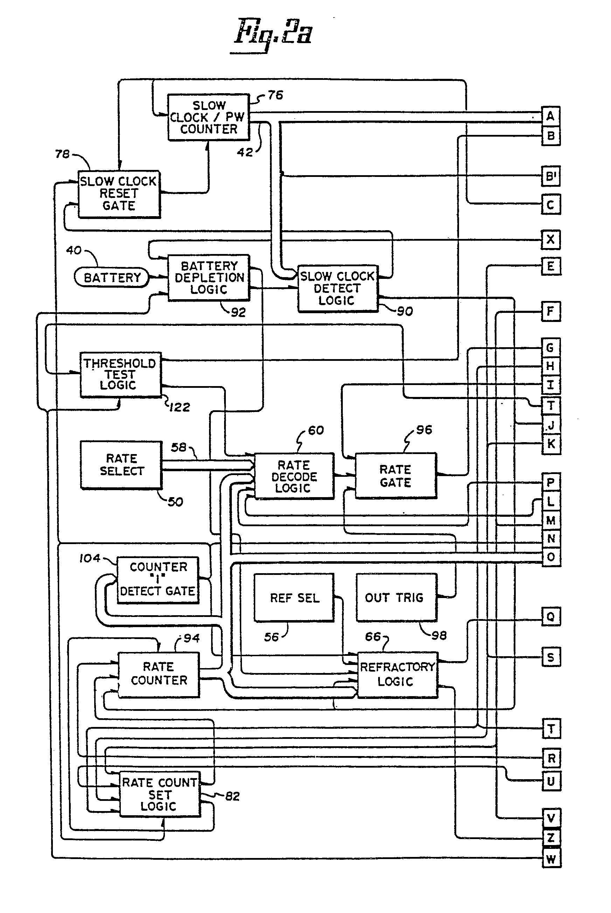

The digital circuit 12 includes the mode selection circuitry, the digital logic necessary to produce each of the selectable output modes, and digital timing means for causing pulses to be generated from pulse generator 10 in the selected manner. A more detailed description of digital circuit 12 is given below in the discussion relating to FIGS. 2a and 2b.

Both the digital and analog circuits are powered by battery 22, which may be a conventional lithium-iodide battery generating +V, or approximately 2.8 volts, connected between a source of reference potential, such as ground 24, and the circuits 12 and 14. In the present description a signal at the power supply voltage may be referred to alternatively as a logic "1" or a high signal. A signal at the ground voltage may be referred to alternatively as a logic "0" or a low signal. The digital circuit 12 is connected to ground at 26 and the analog circuit 14 is connected to ground at 28. Switch 32 is a conventional reed switch and may be closed by placing a magnet (not shown) in close proximity to pulse generator 10 in a manner well known in the art. When reed switch 32 is closed a +V volts or a logic "1" REED signal is applied both to digital circuit 12 and analog circuit 14. When the magnet is removed from the vicinity of the pulse generator 10 reed switch 32 opens and a ground, or logic "0" signal is applied to digital circuit 12 and analog circuit 14. The manner in which such signals are applied shall be described in more detail below.

As mentioned above, outputs 16 and 18 communicate with conventional cardiac pacemaker leads to apply the pulse generator signal to the heart. Output 18 may consist of the outer metal casing of pulse generator 10 or it may connect with an electrode wire within a lead system, depending upon the type of cardiac pacemaker lead selected. Output 16 is coupled to analog circuit 14 through a capacitor 34 to prevent DC leakage in the case of some system failures. A pair of diodes 36 and 38 have their anodes coupled together and their cathodes coupled to outputs 16 and 18 respectively. Diodes 36 and 38 function in the conventional manner to prevent damage to the circuitry of pulse generator 10 in the case of large extraneous signals, such as may be caused by electrocautery.

Analog circuit 14 provides the XTAL, VCO, AMP, RATE LIMIT and BATTERY signals to digital circuit 12. Digital circuit 12 provides the VCO ENABLE, BLANK, and RECHARGE signals to analog circuit 14.

The XTAL signal is a square-wave pulse signal occurring at a frequency of 32,768 Hz and the VCO is a square-wave pulse signal having a frequency of 20,000 Hz whenever the voltage of battery 22 is equal to 2.8 volts and decreases approximately proportional to the square of the voltage to about 10,000 Hz when the battery voltage is equal to 2.0 volts. This decrease of the VCO frequency is used, as shall be explained in more detail below, to provide an increase in the pulse width as the voltage from battery 22 decreases, in order to maintain a constant energy of the pulse.

The VCO ENABLE signal provided from digital circuit 12 to analog circuit 14 is normally a logic "1". However, at the time the stimulation pulse is to be provided, the VCO ENABLE signal becomes a logic "0" and the VC is enabled to begin provided pulses. The VCO ENABLE signal remains logic "0" until after the stimulating pulse has been provided, at which time it returns to logic "1" and the VCO becomes disabled.

The AMP signal is provided from the output of the analog QRS sensing amplifier and is normally a logic "1" signal. Each time the QRS amplifier senses a naturally occurring QRS signal, it becomes a logic "0" pulse signal.

The BLANK signal provided from digital circuit 12 is normally a logic "1" which becomes logic "0" for approximately 100 msec following the provision of a stimulating pulse from pulse generator 10 or the sensing of a natural heartbeat. The BLANK signal is used to prevent the QRS sensing amplifier within analog circuit 14 from sensing any signals during the 100 msec time interval and to allow the components within the sensing amplifier circuit to reset themselves after sensing a signal.

The RECHARGE signal is a normally logic "0" which becomes logic "1" for approximately 10 msec after the stimulating pulse has been provided by generator 10. The purpose of the RECHARGE signal is to close a switch in analog circuit 10 to allow an output capacitor to recharge after every output pulse.

The RATE LIMIT signal which is provided from analog circuit 14 to digital circuit 12 is a normally logic "0" signal which becomes logic "1" after the provision of the stimulation pulse for approximately 500 msec to set an upper rate limit of approximately 120 pulses per minute for pulse generator 10. This rate limitation function is used as a backup and safety feature against the possibility of an integrated circuit or crystal oscillator failure that may cause high rate output to occur.

The BATTERY signal applied from analog circuit 14 to digital circuit 12 is a logic "1" signal so long as the voltage provided from battery 22 is above a certain minimum level, for example 2.0 volts, and is a logic "0" signal whenever the voltage from battery 22 falls below the minimum level.

Referring now to FIG. 2, there is shown the manner of arranging FIGS. 2a and 2b to form a block diagram of digital circuit 12. In FIGS. 2a and 2b any signals which are received from, or applied to analog circuit 14 are designated by the particular label for the signal placed within an oval, for example the battery signal just discussed above is shown at 40 in Fig. 2a. The oval labeled "strobe" is an output to portions of the digital circuit which shall be described below. All provisions of power supply voltage or ground coupled to each block have been deleted, although it should be understood that these signals are necessary and should be coupled in a known and accepted manner of designing digital logic circuits. For each of the blocks shown in FIGS. 2a and 2b data signals are shown as being applied to the left side of the block, reset signals are shown as being applied to the bottom of the block set signals are shown as being applied to the top of the block, and output signals are shown as originating from the right side of the block. Arrows pointing into the block indicate a signal applied to the block, whereas arrows pointing out of the block designate a signal originating from the block. Wherever a plurality of lines are transmitted from, or to a particular block circuit, such as a parallel output from a counter or shift register such plurality of lines are represented as wide lines such as at 42 in FIG. 2a.

Turning now to a description of the manner in which the various subcircuits of the pacemaker are connected and interact as shown in FIGS. 2a and 2b, the pulse rate and hysteresis rate selection circuitry is shown at 50, the pulse width selection circuitry is shown at 52, the hysteresis function selection circuit is shown at 54, and the refractory period selection circuit is shown at 56. Eleven pulse rates and two hysteresis rates are selectable. Selecting a rate, as shall be described below, causes a predetermined combination of logic "1" and logic "0" signals to be output from rate selection circuitry 50 and applied to rate decode logic circuitry 60. If the hysteresis function is selected a logic "1" signal is output from hysteresis function selection circuitry 54 and applied to hysteresis logic 64; if the nonhysteresis function is selected a logic "0" signal is output and applied between the same circuits. The selection of the pulse width causes a predetermined · set of logic "0" and logic "1" signals to be output from the pulse width selection circuitry 52 and applied to the pulse width decode logic circuitry 62. Selection of the refractory period causes a predetermined one of either a logic "0" or a logic "1" signal to be output from refractory selection circuitry 56 and applied to refractory logic 66.

The timing of the pulse generator function is provided by the XTAL oscillator and VCO oscillator signal originating from analog circuit outputs 70 and 72 respectively, and having the frequencies described above. The XTAL and VCO outputs 70 and 72 are applied to crystal/VCO select logic 74. The VCO output also communicates directly with output logic 80, rate count set logic 82, and amp reset logic 84. Crystal/VCO select logic 74 selects one of the XTAL or VCO signals and provides it as the upper output signal which is applied to slow clock/PW counter 76 and to the set input of the slow clock reset gate 78. If any of the inputs to crystal/VCO select logic 74 (other than the XTAL and VCO inputs) are a logic "1" the circuitry selects the VCO signal, and at the same time applies a logic "0" signal through its lower output to VCO ENABLE output 86. If all of the inputs (other than the XTAL and VCO inputs) are a logic "0" the XTAL signal is selected and a logic "1" signal is output to VCO ENABLE 86. The upper output of the crystal/VCO select logic, either XTAL or VCO, provides a fast clock signal for the pulse generator circuit. The VCO pulse starts low (logic "0") and is triggered by the XTAL clock pulse with the result that there is an apparent synchronization between the XTAL and VCO clocks.

Slow clock/PW counter 76 is an eightstage binary counter connected in a known manner. The output signal from slow clock 76 is provided to slow clock detect logic 90, the recharge logic 116 and to pulse width decode logic 62. When slow clock/PW counter 76 counts 179 fast clock cycles detect logic 90 reads this count and provides an output signal through its upper output to slow clock reset gate 78, which in turn provides an output signal to rest slowclock/PW counter 76. The resetting of the slow clock/PW counter 76 after each 179 fast clock cycles determines a time interval called slow clock which is equal to approximately 5.49 msec.

Slow clock detect logic 90 provides a slow clock signal through its lower output to rate outer 94, which is an eightstage binary counter connected in a known manner. The output of rate counter 94 is provided to rate decode logic 60, counter "1" detect gate 104, refractory logic 66, blanking logic 106, and recharge logic 116. The count of rate counter 94 is read by rate decode logic 60 and when the number of counts determined by the inputs to rate decode logic from rate select 50 and hysteresis logic 64 is reached, rate decode logic 60 provides an output signal to rate gate 96. The output from rate gate 96 is provided to crystal/VCO select logic 74, in order to turn on the VCO oscillator as described above, and to output logic 80 in order to initiate the pulse output sequence as shall be described below. Output trigger 98 is a test pin by means of which an external output can be applied to rate gate 96 to force the pulse generator into rate limit either to test the rate limit circuitry and/or in order to perform a laser trim of a resistor on the rate limit circuitry at the time of manufacture.

Output logic 80 is responsive to signals from the pulse width gate 100, and the outputs of rate gate 96 and VCO output 72. The reset input of output logic 80 is responsive to the rate limit output 112 from analog circuit 14. The upper output of output logic 80 is applied to crystal/VCO select logic 74, to rate count set logic 82 and to amp disable logic 102. The middle output is applied to crystal/VCO select logic 74, to output 110 to the analog circuit, to rate count set logic 82, to amp disable logic 102 and to the reset input of hysteresis logic 64. The lower output is applied to recharge logic 116 and rate gate 96.

As mentioned above the signal from rate gate 96 activates the VCO oscillator. On the first VCO pulse output logic 80 is clocked and its upper output changes from logic "0" to logic "1". This signal is applied to crystal/VCO select logic 74 to shut off the VCO oscillator during the period in which the pacemaker is in rate limit as shall be discussed further below, and clocks rate count set logic 82 so that its lower output goes to logic "1". This signal is applied to the set input of rate counter 94 to force all its stages to a logic "1" output stage. Counter "1" detects gate 104 detects this "all 1's" condition and changes its output from logic "0" to a logic "1". This signal from counter "1" detect gate is applied to slow clock reset gate 78 to reset slow clock/PW counter 76 and to the reset input of rate count set logic 82.

On the next VCO oscillation output logic 80 is again clocked and its middle output changes from logic "0" to logic "1". The application of this signal to output 110 initiates the pulse generator output in analog circuit 14. The same signal applied to crystal/VCO select logic 74 continues to enable the VCO oscillator for the duration of the output pulse. The signal is also applied to rate count set logic 82 to enable it so that on the next VCO oscillation its upper output goes from logic "0" to logic "1". This signal is in turn applied to rate counter 94 and clocks it to an overflow condition removing the logic "1" status of each of its stages to prepare the rate counter for the next count cycle.

Rate limit output 112 from analog circuit 14 is applied to the reset input of output logic 80 and to crystal/VCO select logic 74. The rate limit function is a secondary one-shot function on a separate die and is used as a backup against the possibility of integrated circuit of crystal oscillator failure which might create high rate output. The signal applied from rate limit output 112 is normally a logic "0", but during an approximately 500 msec period following the output pulse it is a logic "1". The logic "1" signal applied to the reset input of output logic 80 holds the middle output at a logic "0" until the end of the rate limit period, thereby blocking the output pulse during this period. Thus the rate limit output inhibits rates faster than approximately 120 beats per minute and does not allow the output to go to a 2:1, 3:1, etc. divide condition. The circuitry of output logic 80 is such that if an output pulse were to be called for before the rate limit time period has elapsed, the circuitry is held reset. Then, on the next positive clock transition after the rate limit interval runs out and the signal from rate limit 112 returns to logic "0" the output pulse is initiated.

The signal from rate limit 112 applied to crystal/VCO select logic 74 shuts off the VCO oscillator during the period in which the upper output logic 80 is logic "1" and the rate limit circuitry is on. Thus, if the pacemaker is in a runaway condition in which the output pulse is being called for during the rate limit period, the VCO oscillator is held in the OFF condition. This function minimizes current drain if a runaway condition should arise.

As discussed above, just prior to the initiation of the output pulse the rate counter is set to an "all 1's" condition which condition is detected by counter "1" detect gate 104 which thereupon signals slow clock reset gate 78 to reset slow clock/PW counter 76. Slow clock/PW counter 70 then begins increasing its count again in response to VCO oscillator pulses from crystal/VCO select 74. Pulse width decode logic 62 reads the count of slow clock/PW 76, and when it has reached a count determined by the output of pulse width select 52, pulse width decode logic 62 provides a signal to pulse width gate 100. The output of pulse width gate 100 is provided to output logic 80 to enable the output circuitry so that the output pulse is terminated on the next VCO clock cycle.

The lower output of output logic 80 changes from logic "0" to logic "1" at the termination of the output pulse. This signal is applied to the recharge circuitry to enable it so that after a period determined by a signal provided from slow clock/PW counter 76 the output from recharge logic 116 goes from logic "0" to logic "1" to initiate the recharge interval. This signal is applied to the analog circuit through recharge output 118. Approximately 10 msec after the initiation of the recharge interval recharge logic 116 is reset via a pulse from rate counter 94 and thereby resetting the output to logic "0" to terminate the recharge interval.

Battery output 40 from the analog circuit 14 is provided to battery depletion logic 92. Battery depletion logic 90 is also responsive to the lower output of amp disable logic 102 and the lower output of crystal/VCO select logic 74. The upper output of battery depletion logic 92 is provided to refractory logic 66 and the lower output is provided to slow clock detect logic 90. Battery depletion logic 92 is clocked by crystal/VCO select logic 74 each time the VCO is enabled just prior to an output pulse. This timing of the clock signal prevents supply ripple during or for a period after the pulse from prematurely triggering the battery depletion condition. If the battery is depleted, battery depletion logic 92 provides a logic "0" signal at its lower output which is applied to slow clock detect logic 90 to extend the slow clock interval by 11.1 percent to provide a 10 percent slowdown in the pulse rate. At the same time, a logic "1" signal is provided to refractory logic 66, from the lower output of battery depletion logic 92 to ensure a stable refractory interval as described below. The battery depletion logic circuit is latchable; i.e. if the battery signal shows depletion at the time of any clock pulse provided by crystal/VCO select logic 74, then any subsequent clock pulse results in the output which extends the slow clock interval. The logic may be "unlatched" by the signal from amp disable logic 102, which is provided whenever reed switch 32 is closed. If the BATTERY signal no longer is indicating battery depletion at the time that the reed switch is closed then the battery depletion logic resets to its normal output. However, if the BATTERY signal indicates battery depletion at the time that the reed switch is closed then battery depletion logic 92 remains latched.

We now turn to a discussion of the pacemaker circuitry related to the demand function of the pulse generator, i.e. the function that prevents a generated pulse from being output if a natural heartbeat is detected. Amp output 130 from analog circuit 14 is provided as one input to amp reset logic 84. Other inputs to amp reset logic 84 are from VCO output 72 and the upper output of amp disable logic 102. The upper output of amp reset logic 84 is applied as an input to rate count set logic 82, amp disable logic 102, crystal/VCO select logic 74, and as the set input to hysteresis logic 64. The lower output is provided as an input to rate counter 94 and crystal/VCO select logic 74. In normal operation of the pacemaker a detected heartbeat causes a clock signal to be applied to amp reset logic 84 from output 130. If the upper output of amp disable logic 102 is a logic "1" the clock signal from output 130 causes the upper output of amp reset logic 84 to change to a logic "1" state. The logic "1" signal applied to rate count set logic 82 causes rate counter 94 to be set to "all 1's" as described above and the signal to crystal/VCO select logic 74 turns the VCO on. On the first VCO pulse amp reset logic 84 is clocked to provide a logic "1" signal at its second output which is applied to rate counter 94 and crystal/-VCO select logic 74. The signal to the rate counter 94 clocks the rate count stages to an overflow condition, while the signal to crystal/VCO select logic 74 maintains the VCO ON condition. Amp reset logic 84 resets itself at the same time so that the upper output goes to a logic "0" and one VCO pulse later the second output goes to a logic "0" thereby turning off the VCO and readying the amp reset logic for the next cycle triggered by the next natural heartbeat.

Blanking lock 106 is responsive to the lower output of slow clock detect logic 90 and the output of rate counter 94. Its set input is responsive to the output of counter "1" detect gate 104. The upper output of blanking logic 106 is provided to blank output 134 and the lower output is provided to reversion logic 108 and threshold test logic 122. Blanking logic 106 is clocked each time the lower output of slow clock detect logic 90 changes from logic "0" to logic "1". Most of these clock pulses have no effect on the output states of blanking logic 106 because the circuitry has a self-latching loop that causes the output to retain their previous state with each clock pulse. This state is normally a logic "1" for the upper output and also a logic "1" for the lower output. The signal from counter "1" detect gate 104 however sets blanking logic 106 at the beginning of the output pulse and after a natural heartbeat is sensed. This changes the upper output to a logic "0" to initiate the blanking period and also changes the lower output to a logic "0". The SET signal from counter "1" detect gate 104 goes to a logic "0" on the next VCO pulse, however the blanking logic 106 maintains itself latched in the blanking output condition until approximately 100 msec after the initiation of the pulse when the signal from rate counter 94 relatches blanking logic 106 into the "nonblanking" state described above, thereby terminating the blanking interval. During the blanking interval the sensing amplifier in analog circuit 14 is blanked thereby preventing any input to amp reset logic 84 from amp output 130.

Amp disable logic 102 is responsive to the outputs of reed output 140 and reversion logic 108, the lower output of refractory logic 66, the upper output of amp reset logic 84, and the middle and upper outputs of output logic 80. The lower output of amp disable logic 102 is provided to threshold test logic 122 and battery depletion logic 92. The upper output is provided to amp reset logic 84, while the middle output provides a strobe output 142 which is used elsewhere in the digital circuit and shall be described below. The output to strobe 142 is a logic "0" except in two instances: it is a logic "1" in the time period between the two output pulses from output logic 80, a time period of approximately 25 µ sec just at the initiation of the output pulse; it is also a logic "1" during the time in which the upper output from amp reset logic 84 is a logic "1", that is the period during which the rate counter is being set after each detected natural heartbeat, again a very short period. The lower output of amp disable logic 102 is normally a logic "1" but goes to a logic "0" during a time period between the trailing edge of the first strobe pulse after the reed switch 32 is closed until the trailing edge of the first strobe pulse after reed switch 32 is opened. The upper output of amp disable logic 102 is normally logic "1" whenever either the lower output from refractory logic 55 or the output from reversion logic 108 is a logic "0". The upper output is also a logic "0" during the period described above when the lower output is "0", that is during the approximate time period during which the reed switch is closed.

Refractory logic 66 is responsive to the output of refractory select 56, the upper output of battery depletion logic 92, the lower output of slow clock detect logic 90, and to the output of the rate counter 94. The set input of refractory logic 66 is responsive to the output of counter "1" detect gate 104. The upper output of refractory logic 66 is applied to reversion logic 108, while the lower output is applied to amp disable logic 102. The output of counter "1" detect gate 104 goes to logic "1" just prior to the initiation of the output pulse, and just after the detection of a natural heartbeat as described above. This logic "1" signal sets refractory logic 66 causing its upper output to go to logic "1" and its lower output to go to logic "0". As can be seen from a discussion of the amp disable logic 102 and amp reset logic 84 above the logic "0" state of the lower output of refractory logic 66 causes the upper output of amp disable logic to go to a logic "0" which in turn disables amp reset logic 84 from activation of rate count set logic 82. When the rate counter reaches the count which determines the refractory period the input to refractory logic 66 from rate counter 94 goes to a logic "1". The trailing edge of the same slow clock pulse from slow clock detect logic 90 that advanced rate counter 94 to the refractory count, clocks refractory logic 66 so that its upper output goes to a logic "0" while its lower output goes to a logic "1". This terminates the refractory period and allows the amp reset function to operate as discussed above. If the output of refractory select 56 is logic "0" a 325 msec refractory period is selected; if the output is a logic "1", a 400 msec refractory period is selected. As mentioned above, the input to refractory logic 66 from battery depletion logic 92 causes the refractory logic 66 to recognize an earlier count from rate counter 94 in order that the 11 percent slowdown of the slow clock and rate counter after the battery is depleted is offset and a stable refractory interval is obtained.

Reversion logic 108 is responsive to the lower output of blanking logic 106, the upper output of refractory logic 66, and the output of amp output 130. The output of counter "1" detect gate 104 is applied to the reset input of reversion logic 108. The output of reversion logic 108 is applied to amp disable logic 102 as discussed above. The logic "1" signal from counter "1" detect gate 104 resets reversion logic 108 just prior to the initiation of the output pulse or just after a natural heartbeat. The reset signal places a logic "1" signal on the reversion logic 108 output. At the same time the upper output of refractory logic 66 has become a logic "1" also. At the end of a 100 msec blanking interval the lower output of blanking logic 106 becomes a logic "1" as discussed above. With the inputs from both refractory logic and blanking logic being a logic "1" reversion logic 108 is enabled to receive inputs from amp output 130. This enabling period ends when the upper output of refractory logic 66 goes to a logic "0" at the end of the refractory interval. If two pulses from the amp output 130 are received during this reversion interval the output of reversion logic 108 goes to logic "0" disabling the amp reset logic 84 through amp disable logic 102 as discussed above with respect to the refractory function. Since the refractory interval is either 325 or 400 msec the reversion interval is either 225 or 300 msec; therefore the fact that the reversion is effective if two pulses are detected means that any continuous signal of frequency greater than 8.8 Hz or 6.6 Hz (depending upon the refractory period chosen) is rejected by the reversion circuit.

Hysteresis logic 64 is responsive to the output of the hysteresis function select circuit 54, has a set input from the upper output of amp reset logic 84, and has a reset input from the middle output of output logic 86. Both the upper and lower outputs of hysteresis logic 64 are applied to rate decode logic 60. As discussed above, the amp output 130 signals a detected heartbeat the upper output of amp reset logic 84 goes to a logic "1". This signal sets hysteresis logic 64 so that the upper output goes to a logic "1" while the lower output goes to a logic "0". The logic "1" signal is applied to rate decode logic 60 to enable the hysteresis rate. The logic "0" output is applied to rate decode logic 60 to disable all possible pacemaker rates except the two hysteresis rates. Hysteresis logic 64 will remain in this state as long as the heartbeat rate remains above the hysteresis rate, since each natural heartbeat resets rate counter 94 so that the hysteresis count is not reached. If the heartbeat drops below the hysteresis rate the counter reaches the hysteresis count and signals for an output pulse through rate gate 96 as described above. When the output pulse is initiated by the logic "1" signal from the middle output of output logic 80, a logic "1" reset signal is applied to hysteresis logic 64. The signal causes the upper output of hysteresis logic 64 to change the logic "0" state and the lower output to change to a logic "1" state. The logic "0" signal disables the hysteresis rate in rate decode logic 60 while the logic "1" signal enables the "normal" rates in rate decode logic 60 to provide an output signal at the selected rate. This continues until a natural heartbeat is again detected and the cycle repeats. If the output from hysteresis function select 54 is a logic "0" the upper output of hysteresis logic 64 will remain at a logic "0" state while the lower output will remain at a logic "1" state, which disables the hysteresis function. If the output of hysteresis function select 54 is a logic "1" the hysteresis function is enabled and hysteresis logic 64 operates as discussed.

Threshold test logic 122 is responsive to the lower output of blanking logic 106. The reset input of threshold test logic 122 is responsive to the lower output of amp disable logic 102. The upper output of threshold test logic 122 is applied to pulse width decode logic 62 while the lower output is applied to rate decode logic 60. As discussed above the lower output of amp disable logic 102 is normally a logic "1" which signal holds threshold test logic reset. Both outputs are held in the logic "0" state in this situation. The closing of the reed switch 32 places a logic "0" signal on the lower output of amp disable logic 102 coinciding with the trailing edge of the strobe pulse which comes just at the initiation of the output pulse. This signal enables threshold test logic 122. There is no immediate effect on the rest of the pulse generator circuit. A normal output pulse or natural heartbeat (whichever is dictated by the circumstances) occurs. At the end of the 100 msec blanking period following either the normal output pulse or natural heartbeat the lower output of blanking logic 106 changes from a logic "0" to a logic "1". This signal clocks threshold test logic 122 which changes the lower output to a logic "1", while the upper output remains at a logic "0". The logic "1" signal is applied to rate decode logic 60 to enable the 100 pulse per minute rate. The next output pulse therefore occurs in 600 msec, unless the pacemaker rate or the natural heartbeat rate is faster than 100 beats per minute, in which case a pulse or natural heartbeat at this faster rate occurs. After this pulse or heartbeat threshold test logic 122 is again clocked by blanking logic 106. This third clocking again places the lower output in a logic "1" state and also places the upper output in a logic "1" state. The signal from the lower output again stimulates a 600 msec pulse interval, while the signal from the upper output is applied to pulse width decode logic 62 to cause the pulse width to be seventy-five percent of the selected output pulse width. Thus, on closing the reed switch two normal output pulses at a rate of 100 beats per minute or higher are observed followed by a third pulse at the 100 pulse per minute rate, which pulse is seventy-five percent of the "normal" pulse width. After the narrower pulse threshold test logic 122 is again clocked by blanking logic 106, which signal causes the circuit to reset itself and hold itself reset until the reed switch is again closed.

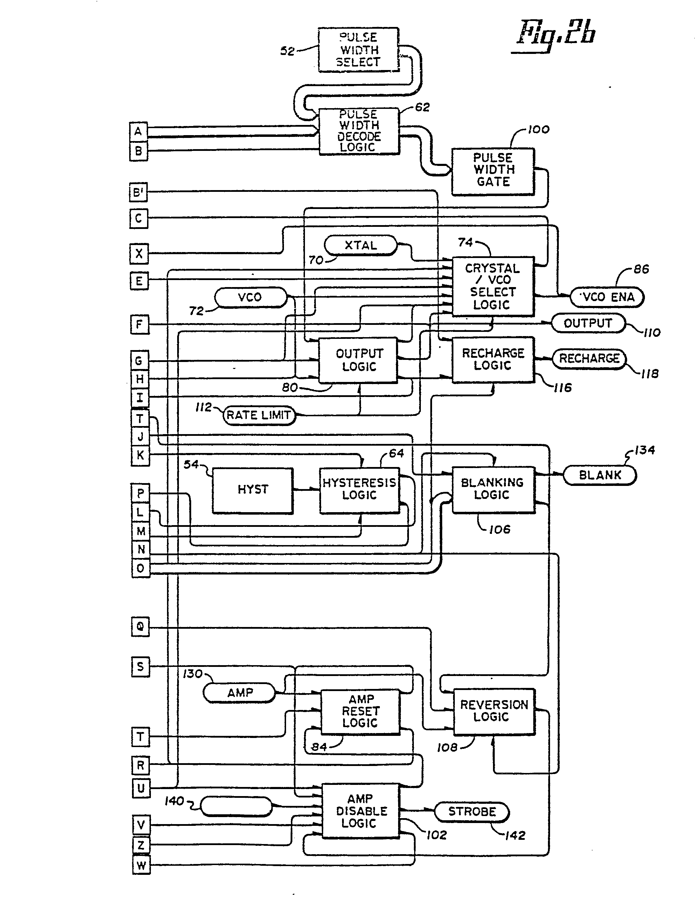

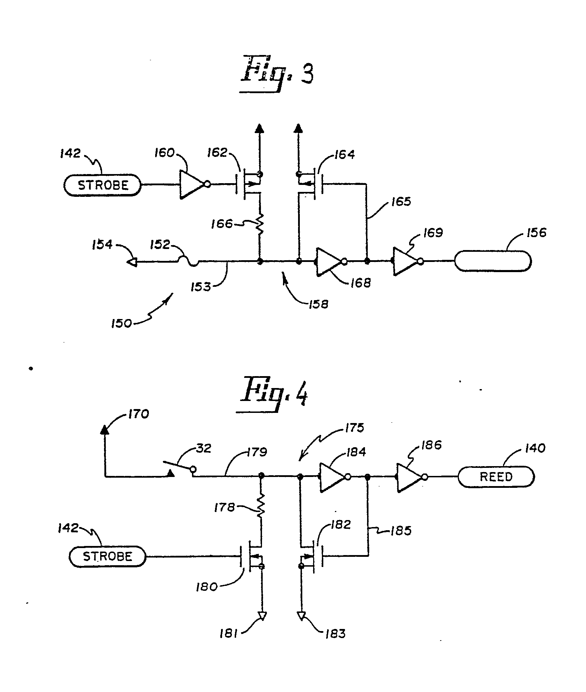

Turning now to FIGS. 3 and 4, the circuitry for selecting the output mode and for setting the mode selection output terminals to a determinate electronic state are shown. FIG. 3 is the embodiment of the circuitry used in connected with the selection of the pulse rate, refractory period, the hysteresis rate, and the hysteresis function. FIG. 4 is the circuit used in selecting the "reed switch mode" that is the mode of operation of the pacemaker when the reed switch 32 is closed.

In FIG. 3 the circuitry for selecting an output mode is shown at 150. This comprises a destructible means 152, a ground 154, and a mode selection output terminal 156. Terminal 156 may be considered to be an output terminal for the circuitry shown in FIG. 3 or an input terminal 50, 52, 54, 56 for the circuitry discussed in connection with the FIGS 2a and 2b. Destructible means 152 connects ground 154 to the circuitry 158 for setting the mode selection input terminals to a determinate electronic state.

The circuitry 158 for setting the mode selection output terminals to a determinate electronic state comprises strobe output 142, inverter 160, p-channel transistors 162 and 164, resistor 166, and inverters 168 and 169. Strobe 142 is applied to the input of inverter 160, while the output of inverter 160 is applied to the gate of fet 162. The input of inverter 168 is connected to one end of fusible link 152 while the output is connected to the input of inverter 169. The output of inverter 169 is applied to terminal 156. P-channel transistor 162 has its source connected to the +V voltage source while its drain is connected to the line between fusible link 152 and inverter 168 through resistor 166. The gate of transistor 164 is connected to the line between the output of inverter 168 and the input of inverter 169. The source is connected to the +V voltage source while the drain is connected to the line between fusible link 152 and the input of inverter 168.

Circuit 158 operates to hold terminal 156 to the logic "0" state when link 152 is conducting, and to hold terminal 156 to a logic "1" state when link 152 is destroyed. If the link 152 is conducting, that is it is not fused, then the line at 153 is a logic "0". Each time strobe output 142 goes to a logic "1" the output of inverter 160 goes to a logic "0" and transistor 162 turns on. However, a voltage drop occurs across resistor 166 and the line at 153 stays low because it is tied directly to ground at 154. The output of inverter 168 is a logic "1" and thus line 165 is high and transistor 164 remains off. Thus, the drain side of transistor 164 remains low which is consistent with line 153 being low. The output of inverter 169 if a logic "0" since its input is a logic "1". Thus, output 156 is held to a logic "0". If link 152 is fused then on the first strobe pulse after it is fused the output of inverter 160 goes to logic "0" and transistor 162 turns on. Since the line at 153 is not longer tied to ground it goes to a logic "1". Since the input of inverter 168 is a logic "1" its output will go to a logic "0" forcing line 165 low and turning transistor 164 on. This causes the drain side of the transistor to go to a logic "1" which is consistent with line 153 being a logic "1". Thus transistor 164 and inverter 168 act as a bistable means. Since the input to inverter 169 is a logic "0" its output is a logic "1" thus output 156 is held in a logic "1" state.

Resistor 166 is preferably about 1—3 megaohms; in actual implementation resistor 166 is not separate from the transistor 162 but rather the transistor is made with a narrow channel which gives the required high impedance. In its preferred embodiment destructible means 152 comprises a metal link preferably comprised of gold. It is either fused by a laser or cut with an abrasive trimmer. It may, however, comprise a metal wire that may be cut, or any other conducting member, the conduction of which may be destroyed by alteration of its physical properties.

The circuit of FIG. 3 is used when the fusible link connects an input with a logic "0" terminal such as ground 154. When the fusible link or other destructible means connects an output terminal such as 156 with a logic "1" terminal then the mirror image circuit would be used. This circuit is shown in FIG. 4. In the present embodiment of the invention the reed switch 32 connects a positive voltage source 170 with the reed input 140 to the digital circuit. Circuit 175 comprises strobe output 142 from the digital circuit, n channel transistors 180 and 182, resistor 178 and inverters 184 and 186. One terminal of reed switch 32 is connected to the input of inverter 184, while the output of inverter 184 is connected to the input of inverter 186. The output of inverter 186 is applied to reed output 140 to the digital circuit. The strobe input 142 from the digital circuit is applied to the gate of transistor 180. The source of transistor 180 is connected to ground while the drain is connected to the line between reed switch 32 and inverter 184 through resistor 178. The gate of transistor 182 is connected to the line between the output of inverter 184 and the input of inverter 186. The source of transistor 182 is again connected to ground while the drain is again connected to a line between reed switch 132 and inverter 184. Resistor 178 is again implemented in this circuit simply by a 1—3 megaohm narrow channel in transistor 180 which provides the required high impedance.

If reed switch 32 is closed line 179 is forced to a logic "1". When strobe 142 turns transistor 180 on there is a voltage drop across resistor 178 accounting for the potential difference between source 170 and ground 181. The line at 185 is a logic "0" due to inverter 184. This maintains transistor 182 in the off condition which is consistent with line 179 being in the logic "1" state. Since the input of inverter 186 is a logic "0" its output will be a logic "1" and thus reed output 140 is held at the logic "1" state. If reed switch 32 is opened, on the first strobe pulse after it is opened transistor 180 is turned on and line 179 becomes a logic "0". The output of inverter 184 thus becomes a logic "1" which turns transistor 182 on which also forces line 179 to a logic "0" latching the circuit. The output of inverter 186 will be a logic "0" since its input is a logic "1", and thus the reed output 140 is held at a logic "0".

A feature of the circuits of FIGS. 3 and 4 are that they permit the terminals 156 and 140 to be held in the required logic "0" or logic "1" state with minimum use of current. Current flows in the circuit of FIG. 3 only for the brief period when strobe 142 is on, and then only if link 152 is not fused. The size of resistor 166 makes even this current minimal. Likewise current flows in the circuit FIG. 4 only when strobe 142 is on and reed switch 32 is closed, and resistor 178 makes this current minimal. As discussed above the strobe pulse has the shortest on time of any signal in the digital circuit and thus the current used by the circuits in FIGS. 3 and 4 is negligible.

In the practice of the invention a pacemaker having circuitry such as is shown in FIGS. 2a and 2b is constructed. Each of the mode selection output terminals of pulse rate and hysteresis rate selection circuitry 50, pulse width selection circuitry 52, hysteresis function selection circuit 54 and refractory period selection circuit 56 are provided with circuitry such as shown in FIG. 3 and the output terminal 140 is provided with input circuitry such as is shown in FIG. 4. There will be a fusible link 152 for each of the above designated terminals of the type in FIG. 3. One or more links are then fused to select the desired output mode of the pulse generator. Preferably the links are arranged together in the circuitry so that they may be considered to correspond to a series of binary digit places. For each model of the pacemaker a binary code is specified and the links corresponding to the binary digit places having a logic "1" value are fused.

The circuits of FIGS. 3 and 4 establish stable voltage values shortly after the links 152 are fused or the reed switch is closed or opened. This permits the fact that the links are fused to be determined with certainty shortly after the fusing is complete so that the operator is working on the circuit for a minimum amount of time and applies a minimum amount of heat in fusing the link which results in minimal contamination of the pacemaker circuitry and a minimum chance of damaging sensitive circuitry elements with the heat. With respect to the reed switch the rapid assumption of the stable state results in more reliable functioning of the digital circuits associated with the reed switch operation.

Since the links may be fused with a minimum of contamination, the chance of failure due to the contaminants is minimized. In such circuits if failure does occur it generally would be due to dendrites growing over the fused ends of link 152, or small conductive dirt such as wire bond chaff or solder balls connecting across the gap due to the production process, static electricity, etc. If such failure does occur the pacemaker output mode will change toward nominal or safe output modes. The reconnection of any of the links 152 corresponding to the rate input will result in a pulse rate no lower than 50 beats per minute; if all the links reconnect then the nominal seventy-beat-per-minute rate is obtained. The reconnection of links 152 corresponding to the pulse width inputs will result in wider pulse widths, which provide more energy and which are more likely to stimulate the heart. The reconnection of the link 152 corresponding to the hysteresis rate will result in the hysteresis rate changing from 60 to 50 beats per minute which allows the heart more opportunity to beat on its own which is generally preferable if a hysteresis function is desirable at all. The reconnection of the link 152 corresponding to input 54 results in the mode going from hysteresis mode to no hysteresis mode, which is preferable since the low hysteresis rates may not be desirable for some patients. If the link 152 corresponding to input 56 reconnects the output mode changes by refractory going from 400 msec to 325 msec which is generally more desirable.

The above description of the invention has been in reference to a particular embodiment. It is evident to those skilled in the art that numerous uses, and modifications of and departures from the specific embodiment described herein may be made without departing from the inventive concepts. For example, those skilled in the art may substitute many other materials for the fusible links including materials whose physical properties may be changed so that the conductivity is destroyed without actual physical separation. Any equivalent electronic parts and electronic circuits may be substituted for those shown. Output parameters other than pulse rate, pulse width, hysteresis and refractory may be programmed using the inventive apparatus. The links may be fused as part of the subassembly of the pacemaker and then the subassembly may be inserted into its place in the pacemaker circuitry. Many other variations may be described.

**Claims**

1. A device for providing electrical stimulation to living tissue, comprising a pulse generator (12) having one or more alterable output parameters, said pulse generator comprising a circuit (60, 62, 64, 66) for determining said output parameter(s), characterised in that said pulse generator further comprises destructible means (152) for selecting said output parameter(s), a destructible means input terminal (154) having a first voltage level, a destructible means output terminal (152), said destructible means being coupled between said destructible means input and output terminals, a transistor (162) having a gate terminal, a source terminal, and a drain terminal, and a strobe means (142) arranged to produce a periodical short pulse coupled to said gate terminal for controlling said transistor, one of said source and drain terminals communicating with an electronic source providing a second voltage level and the other of said source and said drain terminals communicating with said output terminal through a resistance (166) whereby whenever said transistor is turned on by said strobe means while said destructible means is destroyed, said output terminal will assume said second voltage level, and bistable means (164, 168) having an output (156) coupled to an input terminal of said determining circuit and capable of existing in either of two output states and an input communicating with said destructible means output terminal, said output of said bistable means being held in a first output state when said destructible means is intact and said destructible means output terminals is at said first voltage level and said output of said bistable means being held in a second output state when said destructible means is destroyed and said destructible means output terminal is at said second voltage level.

2. A device as claimed in claim 1 wherein said destructible means is a fusible link.

3. A device as claimed in claim 2 wherein said fusible link is composed of gold.

4. A device as claimed in claim 1, 2 or 3 wherein said alterable parameter is the pulse rate of said pulse generator.

5. A device as claimed in claim 4 wherein the pulse rate selected when said destructible means is intact is substantially 70 pulses per minute.

6. A device as claimed in any preceding claim wherein said alterable parameter is the pulse width.

7. A device as claimed in claim 6 wherein the pulse width selected when said destructible means is intact is wider than the pulse width selected when said destructible means is destroyed.

8. A device as claimed in any preceding claim wherein said alterable parameter is the hysteresis function.

9. A device as claimed in claim 8 wherein the hysteresis function is selected when said destructible means is destroyed.

10. A device as claimed in claim 8 or 9 wherein said one or more alterable output parameters further include the hysteresis pulse rate.

11. A device as claimed in any preceding claim wherein said alterable parameter is the refractory period.

12. A device as claimed in claim 11 wherein the refractory period selected when said destructible means is intact is substantially 325 msec.

13. A device as claimed in any preceding claim wherein:
said first voltage level is the ground level; and
said second voltage level is the logic "1" level.

14. A device as claimed in any of claims 1 to 12 wherein:
said first voltage level is a voltage source at the logic "1" level; and
said second voltage level is the logic "0" level.

15. A device as claimed in any preceding claim wherein said bistable means comprises:
a second transistor (164) having a gate terminal, a source terminal and a drain terminal; and
an inverter (168); and
wherein one of said source and drain terminals communicates with said destructible means output terminal, said inverter has its input connected to said one of said source and drain terminals and its output connected to said gate terminal and said other of said source and drain terminals communicates with an electronic source at said second voltage level, and
said output of said inverter is coupled to said input terminal of said determining circuit.

16. A device as claimed in claim 15 wherein said bistable means further comprises a second inverter (169) having its input communicating with the output of said first inverter, the output of said second inverter corresponding to the output of said bistable means.

**Patentansprüche**

1. Gerät zur elektrischen Stimulation von lebendem Gewebe mit einem Impulsgenerator (12), der einen oder mehrere änderbare Ausgangsparameter hat und der eine Schaltungsanordnung (60, 62, 64, 66) zum Bestimmen des oder der Ausgangsparameter aufweist, dadurch gekennzeichnet, daß der Impulsgenerator versehen ist mit einer zerstörbaren Anordnung (152) zur Auswahl des oder der Ausgangsparameter, einem Eingangsanschluß (154) für die zerstörbare Anordnung, der auf einem ersten Spannungspegel liegt, einem Ausgangsanschluß (153) für die zerstörbare Anordnung, die zwischen diese Eingangs- und Ausgangsanschlüsse gekoppelt ist, einem Transistor (162), der einen Gate-Anschluß, einen Source-Anschluß und einen Drain-Anschluß aufweist, und einer Abtastanordnung (142), die einen periodischen kurzen Impuls liefert, der an den Gate-Anschluß zum Steuern des transistors angelegt wird, wobei einer der Source- und Drainanschlüsse mit einer elektronischen Quelle für einen zweiten Spannungspegel in Verbindung steht, während der andere der Source- und Drainanschlüsse mit dem Ausgangsanschluß über einen Widerstand (166) in Verbindung steht, wodurch der Ausgangsanschluß bei zerstörter zerstörbarer Anordnung den zweiten Spannungspegel immer dann annimmt, wenn der Transistor durch die Abtastanordnung eingeschaltet wird, sowie mit einer bistabilen Anordnung (164, 168), die einen an einen Eingangsanschluß der Bestimmungsschaltungsanordnung angekoppelten Ausgang (156), der sich in dem einen oder dem anderen von zwei Ausgangszuständen befinden kann, und einen mit dem Ausgangsanschluß für die zerstörbare Anordnung in Verbindung stehenden Eingang aufweist, wobei der Ausgang der bistabilen Anordnung in einem ersten Ausgangszustand gehalten wird, wenn die zerstörbare Anordnung intakt ist und der Ausgangsschluß für die zerstörbare Anordnung auf dem ersten Spannungspegel liegt, und wobei der Ausgang der bistabilen Anordnung in einem zweiten Ausgangszustand gehalten wird, wenn die zerstörbare Anordnung zerstört ist und der Ausgangsanschluß für die zerstörbare Anordnung auf dem zweiten Spannungspegel liegt.

2. Gerät nach Anspruch 1, wobei die zerstörbare Anordnung ein schmelzbares Zwischenglied ist.

3. Gerät nach Anspruch 2, wobei das schmelzbare Zwischenglied aus Gold besteht.

4. Gerät nach Anspruch 1, 2 oder 3, wobei der änderbare Parameter die Impulsfrequenz des Impulsgenerators ist.

5. Gerät nach Anspruch 4, wobei die bei intakter zerstörbarer Anordnung ausgewählte Impulsfrequenz etwa 70 Impulse/min beträgt.

6. Gerät nach einem der vorhergehenden Ansprüche, wobei der änderbare Parameter die Impulsbreite ist.

7. Gerät nach Anspruch 6, wobei die bei intakter zerstörbarer Anordnung ausgewählte Impulsbreite größer als die Impulsbreite ist, die ausgewählt wird, wenn die zerstörbare Anordnung zerstört ist.

8. Gerät nach einem der vorgehenden Ansprüche, wobei der änderbare Parameter die Hysteresefunktion ist.

9. Gerät nach Anspruch 8, wobei die Hysteresefunktion ausgewählt wird, wenn die zerstörbare Anordnung zerstört ist.

10. Gerät nach Anspruch 8 oder 9, wobei zu dem einen oder den mehreren änderbaren Ausgangsparametern ferner die Hystereseimpulsfrequenz gehört.

11. Gerät nach einem der vorhergehenden Ansprüche, wobei der änderbare Parameter die Refraktärdauer ist.

12. Gerät nach Anspruch 11, wobei die bei intakter zerstörbarer Anordnung ausgewählte Refraktärdauer etwa 325 ms beträgt.

13. Gerät nach einem der vorhergehenden Ansprüche, wobei der erste Spannungspegel der Massepegel ist und der zweite Spannungspegel der LOGISCH-"1"-PEGEL ist.

14. Gerät nach einem der Ansprüche 1 bis 12, wobei der erste Spannungspegel eine Spannungsquelle auf dem LOGISCH-"1"-PEGEL und der zweite Spannungspegel der LOGISCH-"0"-PEGEL ist.

15. Gerät nach einem der vorhergehenden Ansprüche, wobei die bistabile Anordnung aufweist:

einen zweiten Transistor (164) mit einem Gate-Anschluß, einem Source-Anschluß und einem Drain-Anschluß; und

einen Inverter (168); und

wobei einer der Source- und Drain-Anschlüsse mit dem Ausgangsanschluß für die zerstörbare Anordnung in Verbindung steht, der Eingang des Inverters mit dem einen der Source- und Drain-Anschlüsse verbunden ist, der Ausgang des Inverters mit dem Gate-Anschluß verbunden ist und andere der Source- und Drain-Anschlüsse mit einer auf dem zweiten Spannungspegel liegenden elektronischen Quelle in Verbindung steht, und

der Ausgang des Inverters an den Eingangsanschluß der Bestimmungsschaltungsanordnung angekoppelt ist.

16. Gerät nach Anspruch 15, wobei die bistabile Anordnung ferner einen zweiten Inverter (169) aufweist, dessen Eingang mit dem Ausgang des ersten Inverters in Verbindung steht, wobei der Ausgang des zweiten Inverters dem Ausgang der bistabilen Anordnung entspricht.

**Revendications**

1. Dispositif de stimulation électrique de tissu vivant, comportant un générateur d'impulsions (12) ayant au moins un paramètre de sortie modifiable, ledit générateur d'impulsions comprenant un circuit (60, 62, 64, 66) pour déterminer ledit paramètre de sortie, caractérisé en ce que le générateur d'impulsions comprend en outre des moyens destructibles (152) pour choisir ledit paramètre de sortie, une borne d'entrée (154) de moyens destructibles ayant un premier niveau de tension, une borne de sortie (153) de moyens destructibles, lesdits moyens destructibles étant couplés entre les bornes d'entrée et de sortie de moyens destructibles, un transistor (162) comportant une borne de grille, une borne de source et une borne de drain, et un circuit d'échantillonage (142) agencé pour produire une impulsion périodique de courté durée appliquée à ladite borne de grille pour commander le transistor, l'une des bornes de sources et de drain communiquant avec une source d'alimentation électronique fournissant un deuxième niveau de tension et l'autre borne communiquant avec ladite borne de sortie à travers une résistance (166) de manière que même lorsque le transistor est débloqué par les moyens d'échantillonages tandis que les moyens destructibles sont détruit, la borne de sortie reste au second niveau de tension, et un circuit bistable (164, 168) comportant une sortie (156) reliée à une borne d'entrée circuit de détermination et pouvant être à chacun des deux états de sorties et une entrée communiquant avec la borne de sortie de moyens destructibles, la sortie du circuit bistable étant maintenue à un premier état de sortie lorsque les moyens destructibles sont intacts et que la borne de sortie de moyens destructibles est audit premier niveau de tension, et cette sortie de circuit bistable étant maintenue à un second état de sortie lorsque les moyens destructibles sont détruits et que la borne de sortie de moyens destructibles est au second niveau de tension.

2. Dispositif selon la revendication 1, caractérisé en ce que les moyens destructibles consiste en une liaison fusible.

3. Dispositif selon la revendication 2, caractérisé en ce que la liaison fusible est en or.

4. Dispositif selon l'une quelconque des revendications 1 à 3, caractérisé en ce que le paramètre modifiable est la fréquence d'impulsions du générateur d'impulsions.

5. Dispositif selon la revendication 4, caractérisé en ce que la fréquence d'impulsions sélectionnée quand lesdits moyens destructibles sont intacts, est à peu prés de 70 impulsions par minutes.

6. Dispositif selon l'une quelconque des revendications précédentes, caractérisé en ce que le paramètre modifiable est la largeur de l'impulsion.

7. Dispositif selon la revendication 6, caractérisé en ce que la largeur de l'impulsion sélectionnée quand les moyens destructibles sont intacts, est supérieure à la largeur de l'impulsion sélectionnée quand les moyens destructibles sont détruits.

8. Dispositif selon l'une quelconque des revendications précédentes, caractérisé en ce que le paramètre modifiable est la fonction d'hystéresis.

9. Dispositif selon la revendication 8, caractérisé en ce que la fonction d'hystérésis est sélectionnée quand les moyens destructibles sont détruits.

10. Dispositif selon l'une quelconque des revendications 8 ou 9, caractérisé en ce que le au moins un paramètre de sortie modifiable consiste en outre en la fréquence d'impulsions d'hystérésis.

11. Dispositif selon l'une quelconque des revendications précédentes, caractérisé en ce que le paramètre modifiable est la période réfractaire.

12. Dispositif selon la revendication 11, caractérisé en ce que la période réfractaire selectionnée quand les moyens destructibles sont intacts est d'à peu près 325 millisecondes.

13. Dispositif selon l'une quelconque des revendications précédentes, caractérisé en ce que le premier niveau de tension est le niveau de la terre et le second niveau de tension est le niveau logique "1".

14. Dispositif selon l'une quelconque des revendications 1 à 12, caractérisé en ce que le premier niveau de tension est une source de tension au niveau logique "1", et en ce que le second niveau de tension est le niveau logique "0".

15. Dispositif selon l'une quelconque des revendications précédentes, caractérisé en ce que le circuit bistable comprend un deuxième transistor (164) comportant une borne de grille, une borne de source et une borne de drain, et un inverseur (168), et en ce qu'une des bornes de source et de drain communique avec la borne de sortie de moyens destructibles, l'inverseur ayant ses entrées connectées à ladite une des bornes de source et de drain et sa sortie connectées à la borne de grille, et l'autre des bornes de source et de drain communiquant avec une source d'alimentation électronique à un second niveau de tension, et la sortie dudit inverseur étant couplée à la borne d'entrée des moyens de détermination.

16. Dispositif selon la revendication 15, caractérisé en ce que le circuit bistable comprend en outre un second inverseur (169) dont les entrées communiquent avec la sortie du premier inverseur, la sortie du deuxième inverseur correspondant à la sortie du circuit bistable.

_Fig.1_

_Fig.2_

| FIG.2a | FIG.2b |

*Fig.2a*

*Fig.2b*

# Fig. 3

150

142 STROBE — 160 — 162 — 164

154 — 152 — 153 — 166 — 165 — 169 — 156

158 — 168

# Fig. 4

170

32 — 179 — 175 — 184 — 186 — 140 REED

178 — 182 — 185

142 STROBE — 180 — 181 — 183